# EUROPÄISCHE PATENTSCHRIFT

(11) **EP 3 968 879 B1**
(45) Veröffentlichungstag und Bekanntmachung des Hinweises auf die Patenterteilung: **28.02.2024**
(21) Anmeldenummer: 20719365.7
(22) Anmeldetag: 08.04.2020
(51) Int. Cl.: A61B 17/70, A61B 90/00

(54) **MEDIZINISCHER QUERVERBINDER MIT SCHWIMMENDER LAGERUNG**
MEDICAL TRANSVERSE CONNECTOR HAVING A FLOATING BEARING
CONNECTEUR TRANSVERSAL MÉDICAL À PALIER FLOTTANT

(30) Priorität: 17.05.2019 DE 102019113097
(43) Veröffentlichungstag der Anmeldung: 23.03.2022
(73) Patentinhaber: Aesculap AG, 78532 Tuttlingen (DE)
(72) Erfinder: KRÜGER, Sven, 78647 Trossingen (DE)
(74) Vertreter: Winter, Brandl - Partnerschaft mbB
(86) Internationale Anmeldenummer: PCT/EP2020/059961
(87) Internationale Veröffentlichungsnummer: WO 2020/233893

(56) Entgegenhaltungen:
- US-A1- 2008 177 315
- US-A1- 2008 306 538
- US-A1- 2011 137 345
- US-A1- 2013 274 807

## Beschreibung

### Technisches Gebiet

Die vorliegende Offenbarung betrifft einen medizinischen Querverbinder zur Verbindung von zwei vorzugsweise mit Pedikelschrauben oder dergleichen Knochenimplantaten in Wirkeingriff bringbaren Längsstäben, insb. von sogenannten Spinalstäben, wie sie zur Lagepositionierung von Wirbeln einer Wirbelsäule verwendet werden. Ferner betrifft die vorliegende Offenbarung einen zugehörigen Drehmomentschlüssel. Desweiteren wird ein zugehöriges medizinisches Produktset mit dem medizinischen Querverbinder in Kombination mit zumindest einem Knochenimplantat wie einer Pedikelschraube, mit einem chirurgischen Instrument und/oder mit einem anderen passenden chirurgischen Zubehör vorgeschlagen.

Im Stand der Technik der Wirbelsäulenchirurgie ist bekannt, medizinische Querverbinder (englisch: "Cross Connectors") zur Verbindung der Spinalstäbe zum Zwecke der Lagepositionierung, Reponierung, Distraktion, Stabilisierung, etc. von Wirbeln einer, insbesondere menschlichen, Wirbelsäule chirurgisch einzusetzen. Dabei weist der jeweilige Spinalstab üblicherweise zumindest eine Pedikelschraube in Wirkeingriff mit einem Wirbel auf. Dazu wird die Pedikelschraube als Knochenimplantat in einen Pedikel des Wirbels, d.h. in einen Bogenwurzelbereich zwischen dem Wirbelkörper und dem Wirbelbogen, eingeschraubt. Medizinische Indikationen, bei welchen medizinische Querverbinder von einem Anwender wie bspw. einem orthopädischen oder rekonstruktiven Chirurgen bei Operationen an der Wirbelsäule verwendet werden, betreffen degenerative Bandscheibenerkrankungen, Traumata (einschließlich Frakturen oder Dislokationen), posttraumatische Kyphose oder Lordose, Tumoren, Wirbelkörpergleiten, Spinalstenose, Deformitäten (Skoliose, Kyphose und/oder Lordose), Pseudarthrosen nach erfolgloser Wirbelsäulenoperation, symptomatische zervikale Spondylose, Instabilität nach chirurgischem Eingriff aufgrund obiger Indikationen, Reoperationen aufgrund von Versagen einer vorhergehenden Fusion, etc.

Beispielsweise US 2011/137345 A1 offenbart ein interspinöses Befestigungssystem, welches eine Querverbindung, einen ersten Querverbinder und einen zweiten Querverbinder, die jeweils selektiv an Wirbelsäulenfixierungsstäben befestigt werden können, und eine interspinöse Befestigungsvorrichtung mit einem Querverbindungsabschnitt und einen Wirbelsäulen-Befestigungsteil umfasst. Ferner betrifft die US 2011/137345 A1 ein zugehöriges Befestigungsverfahren.

Dabei besteht das Ziel, die seitens des Chirurgen für das Einsetzen des Querverbinders benötigte Zeit weitgehend zu minimieren, um die operative Belastung des Patienten gering zu halten sowie dessen Heilung und Rekreation optimal zu unterstützen. Da insbesondere das Adjustieren des Querverbinders auf die zu behandelnde anatomische Situation und demgemäß auf die dreidimensionale Positionierung der Spinalstäbe sich zeitaufwendig gestaltet, besteht ein Bedürfnis nach Querverbindern, welche sich zügig wie flexibel einsetzen und adjustieren lassen.

So offenbart die EP 1302169 A1 einen medizinischen Querverbinder für zwei Spinalstäbe. Dabei weist ein erstes Anschlussstück des Querverbinders ein erstes Klemmelement und ein zweites Klemmelement und ein Befestigungselement auf, welches dazu ausgelegt ist, das erste Klemmelement und das zweite Klemmelement fest zusammenzuhalten, um so einen Spinalstab zu greifen. Dabei ist wenigstens ein Teil des wenigstens einen mehrachsigen Gelenks zwischen dem ersten Klemmelement und dem zweiten Klemmelement angeordnet, so dass, wenn das Befestigungselement das erste Klemmelement und das zweite Klemmelement zusammenhält, der Teil gequetscht wird, um die Drehung des wenigstens einen mehrachsigen Gelenks zu verhindern. Mit anderen Worten, werden durch Betätigung einer für den Chirurgen zugänglichen Klemmschraube als dem Befestigungselement einerseits die räumliche Stellung des zwischen zwei zu verbindenden Spinalstäben angeordneten mehrachsigen Gelenkes und andererseits einer der zwei Spinalstäbe gleichzeitig fixiert (siehe auch die Figur 3 der EP 1302169 A1). Also werden die Stabklemmung und die Winkellagerung einer Querstrebe des Querverbinders in einem Zug festgestellt.

Jedoch bestehen bei der vorstehend dargelegten Lösung aus dem Stand der Technik einige Nachteile: Zum einen baut die Klemmung des Spinalstabes in hinsichtlich des Patienten anteriorer und posteriorer Richtung auf, was als anatomisch nachteilig bzw. als nicht atraumatisch anzusehen ist. Nicht zuletzt muss somit auch eine Heilungsprognose negativer ausfallen. Zum anderen bedingt die Positionierung der Klemmschraube in direkter Nähe zu dem zu klemmenden Spinalstab eine nachteilige Erweiterung des operativen Zugangs in hinsichtlich des Patienten lateraler Richtung.

Ferner noch besteht der Nachteil, dass durch die parallele Anordnung der beiden unterschiedlichen Klemmungen, also der Klemmung des Spinalstabes einerseits und der Klemmung der Winkellagerung der Querstrebe andererseits, der Korridor eng ausfällt, in welchem beide Klemmungen im selben Zug direkt über die Klemmschraube ausreichend festgestellt werden können. Das zieht den gravierenden Nachteil nach sich, dass die Fertigungstoleranzen der einzelnen mechanischen Elemente des Querverbinders sehr klein gewählt werden müssen. Denn letztlich sind um die beiden unterschiedlichen Klemmungen beide sicher darzustellen, um die Grundfunktion einer auch langzeitstabil sicheren Halterung zu erfüllen. Damit fällt die Fertigung eines solchen bekannten Querverbinders notgedrungen komplex und kostenintensiv aus.

Desweiteren erlaubt der bekannte Querverbinder zwar theoretisch die Klemmung unterschiedlicher Stabdurchmesser, jedoch dürfte eine Variabilität des Stabdurchmessers aus dem oben genannten Zusammenhang der Mechanik der beiden Klemmungen bei dieser Konstruktion eher sehr gering ausfallen.

Somit liegt der Erfindung die Aufgabe zugrunde, einen medizinischen Querverbinder zum Verbinden von zwei beabstandeten Längsstäben eines Knochenimplantats, vorzugsweise Wirbelsäulenimplantats, zu schaffen, welcher die oben dargelegten Nachteile des Standes der Technik überwindet. Zunächst soll ein zügiges, flexibles wie langzeitsicheres Einsetzen und Adjustieren sowohl hin auf die anatomische Situation als auch hin auf unterschiedliche Stabdurchmesser ermöglicht werden. Insbesondere besteht die Aufgabe darin, dem operierenden Arzt einen für ein weitestgehend atraumatisches Einsetzen geeigneten Querverbinder bereitzustellen. Insofern soll der konstruktive Aufbau des Querverbinders einen kleineren operativen Zugangsbereich sowie einen kleineren posterioren Aufbau erfordern, um eine minimale Retraktion bzw. Beeinträchtigung von anatomischen Strukturen wie umliegendem Gewebe oder Nervenwurzeln etc. ermöglichen. Je mehr Strukturen im Zuge des operativen Einsetzens intakt bleiben können, desto mehr wird eine physiologische Umgebung geschaffen, welche die Heilung begünstigt. Die Aufgabe besteht auch darin, eine hinsichtlich benötigter Maßtoleranzen und Passungen des zu fertigenden Querverbinders günstigere Konstruktion zu schaffen.

Diese Aufgabe wird erfindungsgemäß durch die Merkmale des Anspruchs 1 gelöst.

Der medizinische Querverbinder gemäß der vorliegenden Erfindung umfasst eine Querstrebe zur Verbindung zweier vorzugsweise mit Pedikelschrauben oder dergleichen Knochenimplantaten in Wirkeingriff bringbarer Längsstäbe. Die Vorrichtung kann insbesondere zur Lagepositionierung von Wirbeln einer Wirbelsäule dienen. Weiter umfasst der medizinische Querverbinder zumindest einen an der Querstrebe in einer Winkellagerung schwenkbar gelagerten Klemmkopf. Dieser weist eine muldenförmige Stabaufnahme und eine Klemmschraube auf. Dieses Element des Klemmkopfes bzw. Querverbinders dient der Aufnahme und der Klemmung von einem Längsstab derart, dass Längsstäbe unterschiedlichen Durchmessers in flexibler Weise und in Form einer langzeitsicheren Halterung von dem Querverbinder gegriffen werden können. Die Klemmschraube bringt bei deren Betätigung mittels eines über eine Wippenlagerung um eine Schwenkachse schwenkbar gelagerten Klemmzahns eine Klemmkraft auf den zumindest einen in die Stabaufnahme eingesetzten Längsstab auf. Aufgrundessen fixiert die Klemmschraube gleichzeitig oder zeitlich versetzt eine Winkelposition der Winkellagerung zwischen dem Klemmkopf und der Querstrebe. Erfindungsgemäß ist die Wippenlagerung als eine schwimmende Lagerung ausgeführt. Dabei erlaubt die schwimmende Lagerung eine Schwenkbewegung und eine infolge von Klemmkräften zwischen dem Längsstab und dem Klemmzahn erzeugte Lateralbewegung der Schwenkachse des Klemmzahns. Die somit erzeugte Lateralbewegung wirkt durch Umlenken des Kraftflusses auf die Winkelposition der Winkellagerung zwischen dem Klemmkopf und der Querstrebe fixierend und/oder verriegelnd ein.

Also werden erfindungsgemäß über die schwimmende Wippenlagerung die Längsstabklemmung und die Winkellagerung einer Querstrebe des Querverbinders mittels der Betätigung der Klemmschraube nicht in einem Zug, sondern sukzessive bewirkt bzw. festgestellt. Die Fixierung bzw. Verriegelung einer Winkelposition, welche der Klemmkopf und die Querstrebe in dem Moment der Fixierung bzw. Verriegelung zueinander einnehmen, erfolgt infolge der Lateralbewegung, welche ihrerseits infolge von Klemmkräften erzeugt ist.

Dabei kann die Lateralbewegung des Zentrums der Wippenlagerung entlang einer konstruktiv in dem Klemmkopf dazu eingerichteten Wegstrecke erfolgen. Insbesondere ist die die Lateralbewegung führende Wegstrecke entlang der Longitudinalrichtung bzw. einer Longitudinalrichtungskomponente des Klemmkopfes bzw. des Querverbinders ausgerichtet, bspw. entlang einer longitudinal oder schräg angestellt vorgesehenen Langlochbohrung. Dabei kann die Langlochbohrung nicht nur einen linearen, sondern auch einen kurvenförmigen, bspw. einen asymptotischen Verlauf haben. Auch eine besondere Abbildung einer spezifischen Kurvenfunktion über der Longitudinalrichtung ist denkbar.

Mit anderen Worten, werden zum einen die Längsstabklemmung an dem längsstabseitigen Ende des Querverbinders und zum anderen die Winkelung des Querverbinders, an dem der Längsstabklemmung gegenüberliegenden, also an dem ggf. der Wirbelsäule zugewandten, Ende des Querverbinders, sozusagen "hintereinandergeschaltet". Das soll bedeuten, dass die beiden Klemmungen in einem Kraftfluss liegen.

Dies ist durch die schwimmende Ausführung der Lagerung der Schwenkachse (in Transversalrichtung des Querverbinders) des in der Wippenlagerung schwenkbar gelagerten Klemmzahns realisiert. Die Schwenkachse des Klemmzahns erfährt eine Querkraftkomponente zumindest in Longitudinalrichtung des Querverbinders, sobald der Klemmzahn Kontakt zum Längsstab bekommt. Sodann baut sich als Gegenkraft der Kraftwiderstand zum Längsstab je nach dessen Härtegrad bzw. Verformungsfähigkeit und damit resultierender Deformation auf. Diese Querkraftkomponente entspricht also im entstehenden Kräftegleichgewicht derjenigen Kraft, welche zum Klemmen des Stabes führt.

Über die schwimmende Lagerung wird die Gegenkraft dieser Querkraftkomponente der Wippenlagerung, welche an der Schwenkachse des Klemmzahns wirkt, zum Klemm-, Fixierungs- bzw. Verriegelungsmechanismus der Winkellagerung weitergeleitet. Beispielsweise erfolgt die Kraftweiterleitung von der Wippenlagerung hin zu der Winkellagerung über ein Zwischenelement des Klemmkopfes, vorzugsweise über eine U-förmige Gabelhalterung. Dort bewirkt diese Gegenkraft also eine Fixierung bzw. Verriegelung der Winkelung des Klemmkopfes zu der Querstrebe bzw. vice versa in deren Winkellagerung. Insbesondere können im Kräftegleichgewicht zu der Gegenkraft stehende Flächenandruckkräfte entstehen, welche auf Flächenabschnitte der Winkellagerung winkelfixierend und/oder winkelverriegelnd einwirken.

Also ist die Klemmschraube der Wippenlagerung dazu ausgelegt, um in einer ersten Kraftflussphase durch ihr Zustellen den Klemmzahn als ein erstes Klemmmittel in Richtung hin zu dem Längsstab um die Schwenkachse als einer ersten Drehachse zu drehen, bis ein Klemmen des Längsstabes entlang einer Kontaktlinie zwischen Klemmzahn und Längsstab erreicht ist. Und um sodann in einer zweiten Kraftflussphase durch ihr weiteres Zustellen den Klemmzahn um die Kontaktlinie als eine zweite Drehachse zu drehen, um damit einhergehend die Schwenkachse in Richtung weg von dem Längsstab entlang einer Wegstrecke zu bewegen. Dabei ist die Wegstrecke konstruktiv in dem Klemmkopf dazu vorgesehen, eine Lateralbewegung der Wippenlagerung zuzulassen bzw. abzubilden. Somit ist die Wippenlagerung als eine schwimmende Lagerung ausgeführt. Die im Zuge der Einstellung eines Kräftegleichgewichtes an der Wippenlagerung mit gegriffenem Längsstab erzeugte Lateralbewegung baut wiederum die auf die Klemmung der Winkellagerung einwirkenden Klemmkräfte auf. In der Winkellagerung stellt sich ein weiteres Kräftegleichgewicht der Klemmkräfte ein, welches eine Fixierung bzw. Verriegelung der Winkelposition der Winkellagerung bewirkt.

Es ist im Sinne der Erfindung nicht relevant, dass die Längsstäbe eine zylindrische oder quasi-zylindrische Form aufweisen. Der Begriff des Längsstabes ist vorliegend also nicht nur auf längliche Formen mit einem konstanten runden Querschnitt bezogen, sondern umfasst auch jegliche Längsträger mit einem entlang ihrer Längsache veränderlichen Querschnitt und/oder mit einem Querschnitt, welcher eine unrunde Form, bspw. eine ovale, rechteckige, U-, T-, I-förmige, konvexe und/oder konkave Form, aufweist. Im weiteren Sinne kann der Begriff des Längsstabes auch eine Trägerplatte umfassen, vorzugsweise eine Platte, welche entlang zumindest eines Abschnitts ihres Außenumfanges einen einen Halbzylinderstab ausformenden Außenradius ihrer Außenkante aufweist.

Die Unteransprüche zeigen bevorzugte Weiterbildungen der Erfindung.

Vorzugsweise ist die Winkellagerung des medizinischer Querverbinders polyaxial ausgeführt. Das bedeutet, dass die Winkelposition zwischen dem Klemmkopf und der Querstrebe nicht nur uniaxial, also in einer einzigen Achsrichtung, wie bei einem Scharniergelenk als Beispiels eines einachsigen Gelenks der Fall, veränderlich und/oder fixierbar ist. Sondern die Winkelposition ist in dieser bevorzugten Ausführungsform in zumindest zwei, vorzugsweise in drei Achsrichtungen veränderlich und/oder fixierbar. Die von der polyaxialen Winkellagerung eingenommene Winkelposition nach zwei oder drei Ebenen im Raum bestimmt die letztlich zu fixierende bzw. zu verriegelnde Winkelung zwischen dem Längstab und der Querstrebe, um somit mehr räumliche Stellungen des Längsstabes und der Qerstrebe zueinander zu erlauben. Das bewirkt den Vorteil einer gegenüber der anatomischen Situation besser und flexibler anpassbaren Adjustierbarkeit des Querverbinders. Das Entstehen ungünstiger Verspannungen bzw. Verkantungen im Querverbinder bzw. im umgebenden Gesamtsystem, in welches der Querverbinder eingesetzt wird, werden somit besser vermieden.

Vorzugsweise ist ein dreiachsiges Gelenk in Form einer Kugelgelenk-Winkellagerung zwischen dem Klemmkopf und der Querstrebe ausgeführt. Ein dreiachsiges Gelenk besitzt drei Freiheitsgrade hinsichtlich der drei Achsrichtungen der Winkellagerung, nämlich in Longitudinal-, Vertikal- und Transversalrichtung, und zwar jeweils in positiver und/oder in negativer Achsrichtung. Eingeschränkt wird die im Grunde unbegrenzte Beweglichkeit des Kugelgelenks lediglich durch die konstruktiven Elemente der Winkellagerung, welche die Lagerungsfunktion ausführen bzw. eine Führung bewirken. Eine solche besonders bevorzugte Ausführungsform als dreiachsiges Gelenk erlaubt eine Winkelung nach idealerweise nahezu vollständigen drei Freiheitsgraden. Damit sind innere Ausgleichsbewegungen, betreffs des Querverbinders selber bzw. des umgebenden Gesamtsystems, in allen drei Ebenen des Raumes ermöglicht, bevor die Feststellung der Winkelposition durch den Anwender wie einen Chirurgen erfolgt.

Vorzugsweise ist die polyaxiale Winkellagerung mittels eines Federelementes bzw. Vorspannelementes vorgespannt und/oder vorspannbar ausgeführt. Dabei betrifft eine bevorzugte Ausführungsform des Federelementes eine integral in einem Bauteil der Wippenlagerung ausgebildete Zug- und/oder Druck-Feder. Vorzugsweise ist dabei das Bauteil der Wippenlagerung das Zwischenelement des Klemmkopfes, besonders bevorzugt die U-förmige Gabelhalterung. Alternativ und/oder kumulativ ist das Federelement vorzugsweise eingerichtet, zum Klemmen des Längsstabes ein vorangehendes Schnappen auf den Längsstab zu bewirken.

Vorzugsweise ist die Klemmschraube reversibel lösbar. Das ermöglicht dem Anwender ein Nachjustieren nach einem vorläufigen Klemmen. Alternativ oder kumulativ ist die Klemmschraube als selbstsicherndes Befestigungslement ausgeführt. Das sichert die Klemmungen zusätzlich gegen ein ungewolltes Lösen und damit in besonderem Maße gegen ein unerwünschtes Versagen des Querverbinders.

Vorzugsweise ist der Klemmzahn für unterschiedliche Durchmesser des Längsstabes unterschiedlich ausgeformt und/oder dimensioniert einrichtbar und/oder eingerichtet. Dabei ist es weiter bevorzugt, dass der Klemmzahn für Durchmesser des Längsstabes im Intervall von 3 bis 7 mm, besonders bevorzugt für den zervikalen bzw. den Hals betreffenden Wirbelbereich im Intervall von 3,5 bis 4,0 mm und/oder für den lumbalen bzw. die Lendenwirbel betreffenden Wirbelbereich im Intervall von 5,5 bis 6,0 mm eingerichtet ist. Das heisst, dass unterschiedliche Klemmzähne modulartig im Klemmkopf einrichtbar sind. Auf diese Weise kann die Flexibilität des Querverbinders auf spezifische Notwendigkeiten gemäß der anatomischen Gesamtsituation bzw. des Gesamtsystems noch weiter erhöht werden.

Vorzugsweise bestehen Bauteile bzw. Elemente des Querverbinders und/oder der Längsstab aus einem biokompatiblen Material wie einer Keramik. Besonders bevorzugt besteht zumindest eines der oder alle Bauteile bzw. Elemente des Querverbinders und/oder der Längsstab aus einer Titanlegierung. Der Längsstab kann aus einem weichen und/oder harten Material ausgebildet sein.

Als Teil eines medizinischen Produktsets wird ein Drehmomentschlüssel, welcher passend auf eine Klemmschraube eines erfindungsgemäßen medizinischen Querverbinders eingerichtet ist, vorgeschlagen.

Die folgend beschriebenen Merkmale des Drehmomentschlüssels werden nicht beansprucht. Sie dienen lediglich dem Verständnis der Erfindung.

Der zugehörige Drehmomentschlüssel ist einrichtbar und/oder eingerichtet, ein minimal und/oder maximal zulässiges Drehmoment aufzubringen. Dies erfolgt, so dass die auf den zumindest einen in die Stabaufnahme eingesetzten Längsstab aufgebrachte Klemmkraft eine minimal zulässige erste Kraftgrenze nicht unterschreitet und/oder eine maximal zulässige zweite Kraftgrenze nicht überschreitet. Alternativ oder kumulativ erfolgt dies, so dass die aufgrund der auf die Winkelposition der Winkellagerung zwischen dem Klemmkopf und der Querstrebe fixierend bzw. verriegelnd einwirkenden Lateralbewegung bewirkte Gegenkraft eine minimal zulässige dritte Kraftgrenze nicht unterschreitet und/oder eine maximal zulässige vierte Kraftgrenze nicht überschreitet. Alternativ oder kumulativ beträgt das Drehmoment zum Anziehen der Klemmschraube zur Verbindung der Längsstäbe im zervikalen bzw. den Hals betreffenden Wirbelbereich vorzugsweise 2,3 bis 3,3 Nm, besonders bevorzugt 2,8 Nm, und/oder im lumbalen bzw. die Lendenwirbel betreffenden Wirbelbereich vorzugsweise 4,5 bis 5,5 Nm, besonders bevorzugt 5,0 Nm. Der Drehmomentschlüssel kann zur Aufnahme eines Hebelgriffes bzw. Schraubendrehers mit einem Innengewinde bzw. Schrauben-Mitnahmeprofil bspw. in Form eines Sechskants oder eines Innensechsrunds bzw. eines Schrauben-Mitnahmeprofils in Vielrundform (auch bekannt als: "Torx"^{®}) ausgebildet sein.

Ein solcher Drehmomentschlüssel weist den besonderen Vorteil auf, dass herstellerseitig sichergestellt wird, dass die von dem Anwender über die Betätigung der Klemmschraube auf die Wippenlagerung und dann auf die Winkellagerung einzustellenden Klemmkräfte innerhalb vorteilhafter Grenzen bzw. Intervalle zu liegen kommen. Der Einfluss der Handkraft eines individuellen Anwenders wie eines Operateurs auf die Stabklemmung sowie auf die Fixierung bzw. Verriegelung der Winkelposition werden durch den Einsatz eines passenden Drehmomentschlüssels ausgeschaltet bzw. eine personenabhängige Streuung vermieden.

Vorzugsweise ist dabei der zugehörige Drehmomentschlüssel für unterschiedliche Durchmesser des Längsstabes unterschiedlich, vorzugsweise in voreingestellten Intervallen, einstellbar und/oder eingerichtet. Dies erlaubt dem Anwender eine individuelle, aber dennoch herstellerseitig angeleitete, intuitive Einstellung des gewünschten Drehmomentes und der daraus resultierenden Klemmkräfte.

Weiterhin wird ein medizinisches Produktset, vorzugsweise ein Pedikelschrauben- und -hakensystem zur Stabilisation bzw. Korrektur der Wirbelsäule, mit einem erfindungsgemäßen medizinischen Querverbinder vorgeschlagen. Dabei besteht eine Kombination des medizinischen Querverbinders mit zumindest einem Längsstab, vorzugsweise eine Vielzahl von Längsstäben, vorzugsweise in einer Zusammenstellung der Längsstäbe von gerader und/oder gebogener Form und/oder nach unterschiedlichen Größen und/oder Härtegraden und/oder Materialien; und/oder einem Knochenimplantat wie einer Poly- und /oder Monoaxialschraube; und /oder einem Pedikelhaken; und /oder einem chirurgischen Instrument wie einer Stabeindrückzange, einem Implantathalter, einem Distraktor oder dergleichen. Ein solches Produktset wird von behandelnden Ärzten und dem vorbereitenden Klinikpersonal als besonders nützlich angesehen. Insbesondere unterstützt ein solches Produktset eine maßgeschneiderte Operationsvorbereitung. Desweiteren ergeben sich Vorteile für die logistischen Abläufe im herstellerseitigen wie auch im klinischen Bereich.

Das Produktset umfasst weiterhin einen zugehörigen Drehmomentschlüssel.

Abschließend sei darauf hingewiesen, dass der erfindungsgemäße medizinische Querverbinder nicht auf die Verwendung alleinig bei der spinalchirurgischen Lagepositionierung von Wirbeln einer Wirbelsäule mittels Spinalstäben beschränkt ist. Die Erfindung ist gleichermaßen für ähnliche medizinische Verwendungen vorteilhaft einsetzbar, insbesondere für die Vielfalt von chirurgischen, kieferchirurgischen, sportmedizinischen, orthopädischen, rekonstruktiven, kurativen und/oder rehabilitativen Situationen und Maßnahmen. Insbesondere liegen der Frakturbehandlung in den Extremitäten, bspw. bei einem gebrochenen Bein oder Fuß, und der Wirbelsäulenversteifung die gleichen medizinischen Grundprinzipien und therapeutischen Ziele zugrunde.

Insofern umfassen die vorliegenden Begriffe des Längsstabes bzw. des entsprechenden Querverbinders alle dazu medizinisch bzw. spezifisch sinnhaften Ausgestaltungen und/oder Größendimensionierungen und/oder Materialien. Beispielsweise ist im Bereich der plastisch-ästhetischen und/oder rekonstruktiven Kieferchirurgie bzw. Kieferorthopädie von einem vergleichsweise kleineren Maßstab des Längstabes (im Falle eines Spinalstabes hat ein solcher einen bevorzugten Durchmesser von 3 bis 7 mm) bzw. des entsprechenden erfindungsgemäßen Querverbinders auszugehen. Demzufolge ist eine bevorzugte, nämlich insbesondere im Maßstab auf die andere anatomische Situation angepasste bzw. dimensionierte, insb. eine auf den vorliegenden Verhältnisfaktor miniaturisierte, Ausführungsform nicht auszuschließen, sondern von der vorliegenden Offenbarung umfasst.

Der Schutzbereich der vorliegenden Erfindung ist durch die Ansprüche gegeben und wird durch die in der Beschreibung erläuterten oder in den Figuren gezeigten Merkmale nicht beschränkt.

### Kurzbeschreibung der Figuren

Fig. 1 ist eine (leicht perspektivische) Vorderansicht gemäß einem ersten Ausführungsbeispiel des medizinischen Querverbinders nach der vorliegenden Offenbarung, in welchen ein Längsstab eingeklemmt ist;
Fig. 2 ist eine erste perspektivische Vorderansicht gemäß dem ersten Ausführungsbeispiel des medizinischen Querverbinders, zur Veranschaulichung des über die Wippenlagerung schwenkbar gelagerten Klemmzahnes ohne Gehäuse des Klemmkopfes dargestellt;
Fig. 3 ist eine Aufsicht gemäß dem ersten Ausführungsbeispiel des medizinischen Querverbinders, insb. den Kraftfluss von der schwimmenden Wippenlagerung zu der Winkellagerung veranschaulichend;
Fig. 4 ist eine zweite perspektivische Vorderansicht gemäß dem ersten Ausführungsbeispiel des medizinischen Querverbinders (ohne Längsstab).
Figuren 5a bis 5c zeigen jeweils eine Aufsicht gemäß einem zweiten Ausführungsbeispiel des medizinischen Querverbinders in einer funktionalen Abfolge zu drei Zeitpunkten [a) offen; b) stabklemmend; c) winkelfixiert], insb. veranschaulichend die Funktionsweise der auf die Winkelfixierung der Winkellagerung einwirkenden schwimmenden Wippenlagerung eines einen ersten Längsstab klemmenden Klemmzahns.
Figuren 6a bis 6c zeigen jeweils eine Vorderansicht im Längsschnitt gemäß dem zweiten Ausführungsbeispiel des medizinischen Querverbinders zu den jeweiligen drei Zeitpunkten der vorgenannten Figuren 5a bis 5c.
Fig. 7 ist eine Vorderansicht im Längsschnitt gemäß dem zweiten Ausführungsbeispiel des medizinischen Querverbinders zu dem dritten Zeitpunkt eines winkelfixierten (winkelverriegelten) Zustandes anhand eines, im Vergleich zu dem ersten Längsstab in Fig. 6c dickeren, zweiten Längsstabes.
Fig. 8 ist ein Detail einer Vorderansicht im Längsschnitt gemäß einer bevorzugten Abwandlung des zweiten (bzw. des ersten) Ausführungsbeispieles der vorliegenden Erfindung betreffs einer federnden Ausgestaltung der Gabelhalterung.
Figur 9 ist eine Perspektivenansicht einer mittig gekoppelten Anordnung von zwei Querverbindern gemäß dem zweiten Ausführungsbeispiel.
Figur 10 ist eine Vorderansicht im (Teil-)Längsschnitt einer mittig gekoppelten Anordnung von zwei Querverbindern gemäß dem zweiten Ausführungsbeispiel.

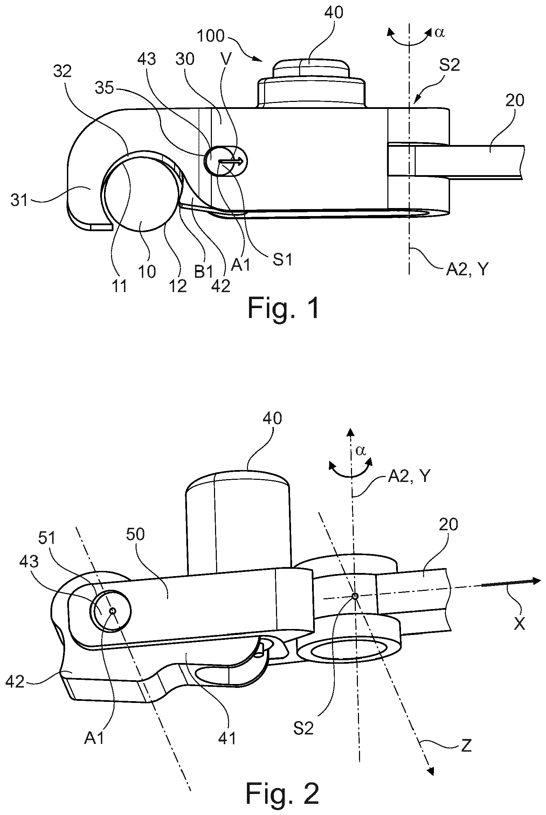

Nachstehend wird ein erstes Ausführungsbeispiel der vorliegenden Offenbarung auf der Basis der zugehörigen Figuren 1 bis 4 beschrieben. Daraus ergeben sich weitere Einzelheiten, Merkmale und Vorteile der Erfindung.

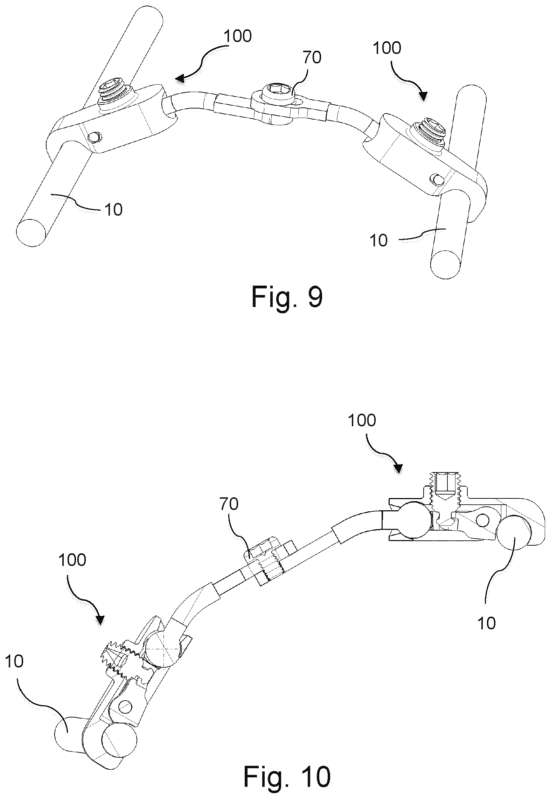

Figuren 1 bis 4 zeigen unterschiedliche Ansichten gemäß einem ersten Ausführungsbeispiel eines erfindungsgemäßen medizinischen Querverbinders 100. Fig. 1 zeigt eine leicht perspektivische Vorderansicht des medizinischen Querverbinders 100, in welchen ein Spinalstab 10 als Längsstab (links in Fig. 1) eingeklemmt ist. Der medizinische Querverbinder 100 umfasst zunächst eine Querstrebe 20 (rechts in Fig. 1) zur medialen Verbindung zweier Spinalstäbe 10, wovon der zweite nicht von der Darstellung umfasst ist. Zumeist (nicht dargestellt) sind zwei Querverbinder 100 hinsichtlich des Patienten medial, d.h. über der Wirbelsäulenmitte, miteinander zu einer Anordnung gekoppelt (siehe zum Vergleich auch Figuren 9 und 10 für das zweite Ausführungsbeispiel). Insofern sind die Ansichten aller Figuren 1 bis 4 unterbrochen, was durch die Wellenlinie gemäß der Darstellungsnorm für technische Zeichnungen (rechts in Figuren 1 bis 4) angedeutet ist. Dadurch wird über die Wirbelsäulenmitte hinweg in hinsichtlich des Patienten lateraler Richtung, also quasi in Rippenrichtung, überbrückt. Auch ist dann der einzelne Querverbinder in ein die Wirbelsäule stabilisierendes Gesamtsystem zur Lagepositionierung der einzelnen Wirbel eingebunden. Jeweilige Spinalstäbe 10 oder Trägerplatten werden im Zuge einer wirbelsäulenchirurgischen Operation mittels Pedikelschrauben in Weise eines Knochenimplantats zu den Wirbeln in Wirkeingriff gebracht (nicht dargestellt).

Desweiteren umfasst der Querverbinder 100 einen gehäuseartig ausgeformten Klemmkopf 30 mit einer muldenförmigen Stabaufnahme 31 (links in den Figuren 1, 3 und 4; an der lateralen Kopfseite des Querverbinders 100 vorgesehen). Die muldenförmige Stabaufnahme 31 weist eine innere Lagerfläche 32 auf, die in etwa mit einer zu einem zu greifenden Spinalstab 10 komplementären Form ausgebildet ist. Hier ist die innere Lagerfläche 32 mit einem im Vergleich zum Radius des Spinalstabes 10 etwas weiteren Radius kelchartig auslaufend ausgebildet und wie ein Hakenmaul ausgelegt, an einem zylindrischen äußeren ersten Oberflächenabschnitt 11 des Spinalstabes 10 anzugreifen. An einem in etwa diametral gegenüberliegenden zweiten Oberflächenabschnitt 12 des Spinalstabes 10 greift als Gegenelement ein Klemmzahn 42 an. Dazu ist der Klemmzahn 42 über eine in dem Klemmkopf 30 vorgesehene Wippenlagerung S1 mit einer Schwenkachse A1 um diese schwenkbar gelagert. Zur Halterung des Spinalstabs 10 kann der Klemmzahn 42 an diesen durch eine Schwenkbewegung (in Fig. 1: im Uhrzeigersinn) um die Schwenkachse A1 angedrückt werden, wodurch eine Kontaktlinie B1 zwischen der konvexen Spitze des Klemmzahns 42 und dem zweiten Oberflächenabschnitt 12 des Spinalstabes 10 ausgebildet wird. Hier liegt die Kontaktlinie B1 im wesentlichen parallel zur Schwenkachse A1 vor.

Zur Ausbildung der Schwenkachse A1 ist ein Zylinderbolzen 43 in Transversalrichtung Z in zwei, auf gegenüberliegenden Seitenflächen des Klemmkopfs 30 vorgesehenen, Langlochbohrungen 35 aufgenommen. Aufgrund der Lagerung des Zylinderbolzens 43 in den Langlochbohrungen 35 ist die Wippenlagerung S1 als schwimmende Lagerung ausgeführt. Insofern ermöglicht die schwimmende Lagerung eine Relativbewegung bzw. eine Ausweichbewegung des die Schwenkachse A1 abbildenden Zylinderbolzens 43 entlang der Langlochbohrungen 35 zur Beschreibung eines Lateralbewegungsvektors V der Schwenkachse A1 in der Wippenlagerung S1.

Die innere Winkelung des Querverbinders 100 ist in dem Freiheitsgrad eines Scharniergelenks S2 als uniaxialer Winkellagerung S2 (rechts in den Figuren 1 bis 4) veränderlich. Dazu ist in dem Scharniergelenk S2 der Klemmkopf 30 an der Querstrebe 20 schwenkbar gelagert. Die die Winkelung zwischen zentralen Körperachsen des Klemmkopfes 30 und der Querstrebe 20 als Winkelschenkeln bestimmende Winkelposition α ist um eine einzige Gelenkachse A2 als Drehachse in dem Scharniergelenk S2 veränderlich. In dem in Fig. 1 gezeigten Beispiel fällt die Gelenkachse A2 mit der Vertikalrichtung Y des Klemmkopfes 30 zusammen. Somit ist die Winkelposition α in einer von der Longitudinalrichtung X und der Transversalrichtung Z (siehe Fig. 2) aufgespannten Ebene verschwenkbar.

Desweiteren umfasst der Querverbinder 100 eine Klemmschraube 40 (oben in Fig. 1). Die Klemmschraube 40 dient der Betätigung der Klemmmechanismen des Querverbinders 100 durch einen Anwender, bspw. durch einen Wirbelsäulenchirurgen von posteriorer Seite. Zunächst wird durch Zustellung der Klemmschraube 40 ein Befestigen des Querverbinders 100 an dem Spinalstab 10 mittels des schwenkbar gelagerten Klemmzahnes 42 bewirkt. Und im Weiteren wird durch Umlenken des Kraftflusses ein Feststellen einer inneren Winkelung des Querverbinders 100, d.h. der Winkelposition α, bewirkt. Zwischendurch kann der Anwender das Scharniergelenk S2 auf eine richtige, spannungsfreie bzw. gewünschte Lage noch einmal genauer ausrichten bzw. nachjustieren und mittels einer Weiterbetätigung der Klemmschraube 40 eine aktuelle Winkelposition α fixieren bzw. verriegeln. Insgesamt kann der Anwender in einem durchgängigen bzw. nur kurz unterbrochenen Feststellvorgang die Klemmschraube 40 weiter, idealerweise moderat, in den Klemmkopf 30 hineinschrauben, nachdem er zunächst die Klemmung des Spinalstabes 10 bereits bewirkt hat.

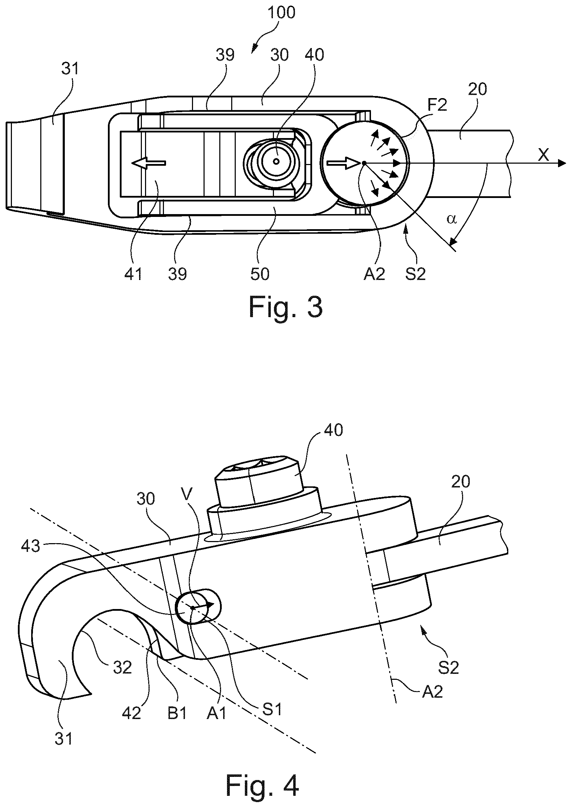

Dazu erlaubt die schwimmende Wippenlagerung S1 eine(n) infolge von Klemmkräften zwischen dem Längsstab 10 und dem Klemmzahn 42 erzeugte(n) Lateralbewegung(svektor) V der Schwenkachse A1 des Klemmzahns 42, welche(r) durch die Langlochbohrung 35 geführt bzw. begrenzt ist. Damit geht einher, dass eine Ausweichbewegung der Schwenkachse A1 um die zwischen der konvexen Spitze des Klemmzahns 42 und dem zweiten Oberflächenabschnitt 12 des Spinalstabes 10 ausgebildete Kontaktlinie B1 stattfindet. Mit anderen Worten, drückt sich der Zylinderbolzen 43 über den mittels ihm gelagerten Klemmzahn 42 entlang der Kontaktlinie B1 von dem Längsstab 10 ab, um eine Ausweichbewegung in Form des Lateralbewegungsvektors V entlang der Langlochbohrung 35 zu vollziehen. Der Zylinderbolzen 43 der schwimmenden Wippenlagerung S1 verschiebt sich also um den Lateralbewegungsvektor V, und zwar weg vom Längsstab 10 und in Richtung hin zu dem Scharniergelenk S2. Diese Mechanik wiederum wirkt auf die Winkelposition α des Scharniergelenks S2 zwischen dem Klemmkopf 30 und der Querstrebe 20 fixierend bzw. verriegelnd ein (siehe auch in Figur 3 symbolhaft dargestellte Kraftpfeile).

Figuren 2 und 3 veranschaulichen die konstruktiven Details innen im Klemmkopf 30 des Querverbinders 100, welche das Zusammenspiel bzw. den Kraftfluss innerhalb der schwimmenden Wippenlagerung S1 bis hin zu dem Scharniergelenk S2 umsetzen. Und zwar zeigt Fig. 3 eine Aufsicht gemäß dem ersten Ausführungsbeispiel des medizinischen Querverbinders 100, wobei insb. mittig in die Anordnung eingetragene Pfeile den Kraftfluss der Klemmkräfte andeuten sollen. Wiederum Fig. 2 zeigt eine erste perspektivische Vorderansicht gemäß dem Ausführungsbeispiel des medizinischen Querverbinders 100, welcher im Sinne der Veranschaulichung des über die Wippenlagerung S1 schwenkbar gelagerten Klemmzahnes 42 ohne äußeres Gehäuse des Klemmkopfes 30 dargestellt ist. So ist der Blick freigegeben auf die schwenkbare Lagerung des Klemmzahnes 42, welche mittels des in zwei Zylinderbohrungen 51 auf jeweils gegenüberliegenden Seiten einer U-förmig ausgebildeten Gabelhalterung 50 eingefassten Zylinderbolzens 43 umgesetzt ist. Die Gabelhalterung 50 ist außenseitig zu einem Drückelement 41 des Klemmzahns 42 im Inneren des Klemmkopfes 30 und dazwischen beweglich angeordnet. Dabei ist der Klemmzahn 42 bzw. das Drückelement 41 von der Gabelhalterung 50 an einer von der Stabaufnahme 31 abgewandten und dem Scharniergelenk S2 zugewandten Stirnseite U-förmig geschlossen umfasst. Hingegen ist die kopfseitige Stabaufnahme nicht von der Gabelhalterung 50 umfasst bzw. wird von dieser frei gelassen. Bei einer Bewegung des den Klemmzahn 42 bzw. das Drückelement 41 lagernden Zylinderbolzens 43 entlang dem Lateralbewegungsvektor V wird die Gabelhalterung 50 an ihren äußeren Seitenflächen durch zugehörige Innenflächen 39, 39 des Klemmkopfes 30 in diesem beweglich geführt. Aufgrund des Lateralbewegungsvektors V der Gabelhalterung 50 entstehen in dem Übergang zur Querstrebe 20 im Bereich des Scharniergelenkes S2 winkelfixierende Klemmkräfte, wie sie anhand von dünnen Pfeilen als Flächenandruckkräfte in dem der Querstrebe 20 zugewandten konvexen Flächenabschnitt F2 des Scharniergelenkes S2 angedeutet sind. Im Ergebnis fixiert bzw. verriegelt die Klemmschraube 40 zuletzt die Winkelposition α des Scharniergelenks S2 zwischen dem Klemmkopf 30 und der Querstrebe 20.

Fig. 4 zeigt eine im Vergleich zu der Fig. 1 stärker perspektivische Vorderansicht des medizinischen Querverbinders 100, jedoch ohne einen eingeklemmten Spinalstab 10. Zur Vermeidung von Wiederholungen wird auf die Ausführungen zu Fig. 1 verwiesen.

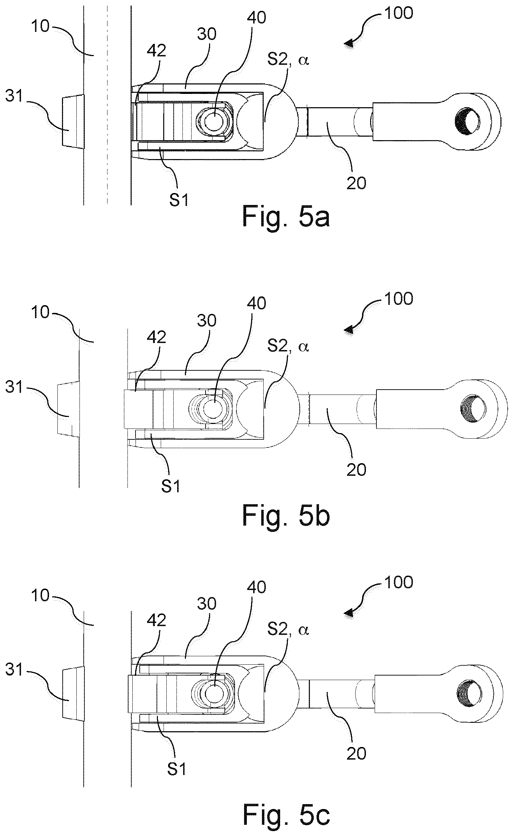

Im Nachfolgenden wird ein zweites Ausführungsbeispiel der vorliegenden Erfindung anhand der Fig. 5a bis 7 beschrieben, wobei im Wesentlichen nur auf die zum ersten Ausführungsbeispiel unterschiedlichen konstruktiven Merkmale eingegangen wird. Bezüglich aller weiteren Merkmale und Funktionsweisen wird auf die vorstehende Figurenbeschreibung verwiesen.

Bei dem zweiten Ausführungsbeispiel ist die aus dem ersten Ausführungsbeispiel eines Querverbinders 100 bekannte, als Scharniergelenk S2 ausgeführte, uniaxiale Winkellagerung S2 durch eine, als Kugelgelenk S2 ausgeführte, polyaxiale Winkellagerung S2 zwischen dem Klemmkopf 30 und der Querstrebe 20 ersetzt.

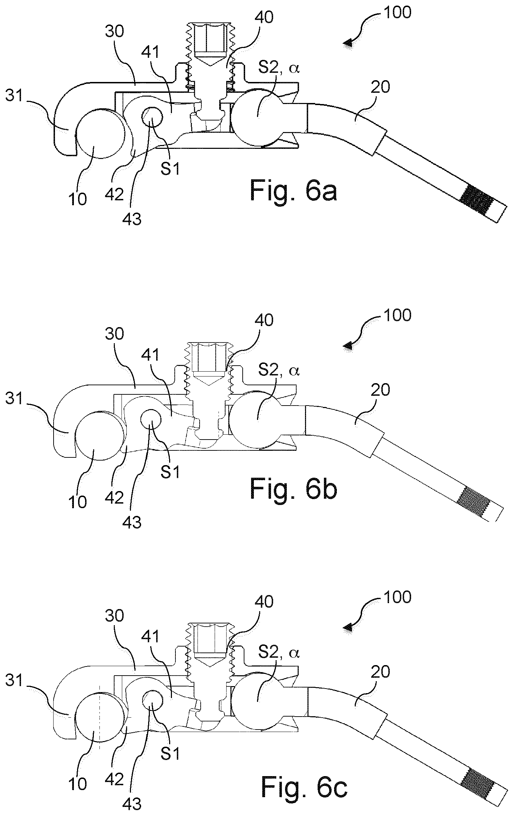

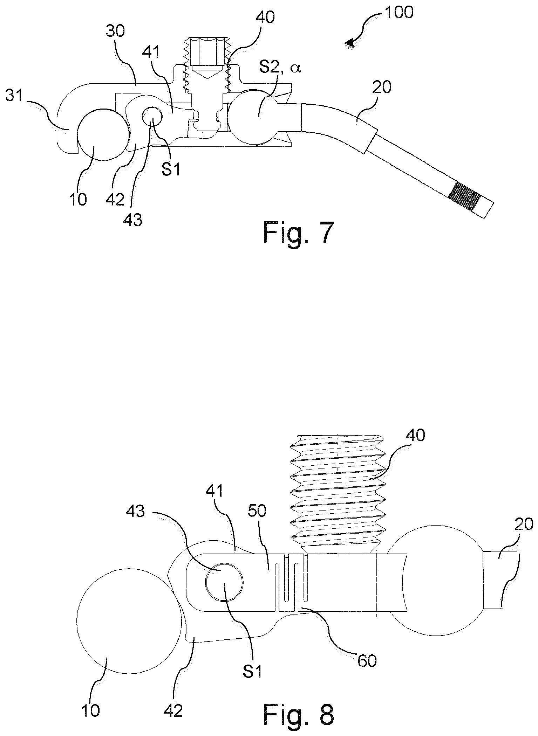

Im Konkreten ist der funktionale Ablauf der beiden zuvor beschriebenen Klemmwirkungen in der Abfolge von jeweils drei zusammenhängenden Figuren 5a bis 5c bzw. Figuren 6a bis 6c veranschaulicht, welche drei unterschiedlichen Zeitpunkten entsprechen. Dabei zeigen die Figuren 5a bis 5c eine Aufsicht auf den Querverbinder 100 von einer der Klemmschraube 40 abgewandten Seite, wobei auch ein eingelegter Spinalstab 10 dargestellt ist. Entsprechend zeigen Figuren 6a bis 6c dieselben drei Zeitpunkte in der Darstellung als ein Längsschnitt durch den Querverbinder 100. Der Spinalstab 10 weist dabei in allen Figuren 5a bis 6c einen vergleichsweise kleineren Durchmesser auf (siehe zum Vergleich Fig. 7 mit einem alternativen Spinalstab 10 eines dazu vergleichsweise größeren Durchmessers). So ist der Mechanismus bzw. die Kinematik der zuvor beschriebenen sukzessiven Klemmwirkungen, zunächst eines in den Querverbinder 100 eingelegten Spinalstabs 10 mittels des in der schwimmenden Wippenlagerung S1 gelagerten Klemmzahnes 42 sowie sodann hinsichtlich der dreidimensional schwenkbeweglichen Winkelposition α in dem Kugelgelenk S2, zu drei markanten Zeitpunkten dargestellt: Und zwar markiert ein erster Zeitpunkt, wie in den Figuren 5a bzw. 6a dargestellt, eine Anfangsstellung vor dem Einschrauben bzw. Zustellen der Klemmschraube 40, in welcher der Spinalstab 10 lose von der Stabaufnahme 31 umgriffen bzw. in diese hineingelegt ist. Ein darauffolgender zweiter Zeitpunkt, wie in den Figuren 5b bzw. 6b dargestellt, markiert eine initiale Klemmstellung. In dieser ist der Klemmzahn 42 durch weitere Betätigung der Klemmschraube 40 zur Verschwenkung des Drückelementes 41 im Uhrzeigersinn in einen ersten Kontakt mit dem Spinalstab 10 gekommen, so dass sich eine Klemmkraft zwischen dem Klemmzahn 42 und dem Spinalstab 10 aufgebaut hat. Ein dritter Zeitpunkt, wie in den Figuren 5c bzw. 6c dargestellt, markiert eine Position, in welcher über das Ausweichen der schwimmenden Wippenlagerung S1 seitwärts weg vom geklemmten Spinalstab 10 ein Kraftfluss zum Verriegelungsmechanismus des Kugelgelenks S2 stattfindet. Dabei übt der Kraftfluss eine winkelfixierende Wirkung auf die räumliche Winkelposition α aus, in welcher sich das Kugelgelenk S2 zu dem Zeitpunkt gerade befindet.

Fig. 7 entspricht der zuvor beschriebenen Fig. 6c zeigt gleichfalls den Querverbinder im Längsschnitt zu dem dritten Zeitpunkt, in welchem die winkelfixierende bzw. winkelverriegelnde Wirkung auf das Kugelgelenk S2 erreicht ist. In Gegenüberstellung zu der Fig. 6c ist in Fig. 7 die finale Situation mit einem alternativen Spinalstab 10 eines dazu vergleichsweise größeren Durchmessers dargestellt. Insofern liegt der in Fig. 7 dickere Spinalstab 10 mit einem zu der Stabaufnahme 31 in etwa komplementären Radius um seinen Querschnittsumfang nahezu vollständig an. Der Vergleich der beiden Figuren verdeutlicht, dass es aufgrund der Ausgestaltungen der Klemmung mittels des in der schwimmenden Wippenlagerung S1 gelagerten Klemmzahnes 42 gut möglich ist, Spinalstäbe 10 unterschiedlicher Durchmesser sicher zu umfassen und zu klemmen und gleichzeitig eine gute winkelfixierende Klemmwirkung auf das Kugelgelenk darzustellen.

In Nachfolgenden wird anhand der Fig. 8 eine bevorzugte Abwandlung des zweiten (bzw. des ersten) Ausführungsbeispieles der vorliegenden Erfindung beschrieben. Dabei ist im Wesentlichen nur auf das zum zweiten Ausführungsbeispiel besonders ausgeführte konstruktive Merkmal eines mittels eines Federelementes 60 vorgespannten und/oder vorspannbaren Kugelgelenkes S2 zu achten. Das Federelement ist in diesem spezifischen Ausführungsbeispiel erkennbar als mechanische Druckfeder integral in die U-förmige Gabelhalterung 50 (siehe zum Vergleich auch Fig. 2) mit mäanderförmiger Kontur eingeschnitten, bspw. mittels einer spanabhebenden Fertigung, eines Säge- oder Drahterosionsverfahrens, etc. Bezüglich aller weiteren Merkmale und Funktionsweisen wird auf die vorstehende Figurenbeschreibung für das zweite Ausführungsbeispiel (Kugelgelenk S2, d.h. polyaxial) bzw. für das erste Ausführungsbeispiel (Scharniergelenk, d.h. uniaxial) verwiesen.

Die Figuren 9 und 10 zeigen eine Anordnung von zwei Querverbindern, welche in dieser Darstellung gemäß des zweiten Ausführungsbeispieles dargestellt sind, um die besonders hohen dreidimensionalen Freiheitsgrade der beiden jeweiligen Kugelgelenke zu veranschaulichen. Figur 9 zeigt eine perspektivische Aufsicht (wie von posterior). Hingegen die entsprechende Figur 10 zeigt einen leicht perspektivischen Längsschnitt. Darin liegt die Blickrichtung parallel zu der Längsachse des rechts, im Querschnitt dargestellten zweiten Spinalstabs 10 (d.h. Längsachse orthogonal zur Papierfläche) bzw. leicht gewinkelt, also nur in etwa parallel zu der Längsachse des links, in einer leicht perspektivischen Aufsicht im Querschnitt dargestellten ersten Spinalstabs 10.

Hinsichtlich der (nicht dargestellten) Wirbelsäule eines Patienten ist die längliche Anordnung der zwei Querverbinder 100 als hinsichtlich des Patienten medial, d.h. über der Wirbelsäulenmitte, gekoppelte Anordnung zu denken. Die mittige bzw. mediale Kopplung der zwei Querverbinder 100 miteinander erfolgt mittels einer Querverbinder-Zentralverbindung 70. Dabei bildet der laterale Verlauf der Anordnung, von dem (links dargestellten) ersten Spinalstab 10 über die beiden Querverbinder 100 hinweg zu dem (rechts dargestellten) zweiten Spinalstab 10, eine anatomisch günstige, gebogene Wölbung über der Wirbelsäulenmitte aus (In Fig. 9 ist die Wirbelsäulenmitte zu denken als unterhalb der Querverbinder-Zentralverbindung 70 angeordnet).

Auch in diesem Fall können sämtliche Vorteile des ersten Ausführungsbeispiels verwirklicht werden. Der Vollständigkeit halber sei jedoch angemerkt, dass sich ein nicht unähnliches Bild für eine Anordnung von zwei Querverbindern des ersten Ausführungsbeispiels ergäbe, wenn auch weniger Freiheitsgrade für die gesamte Winkelung einer solchen Anordnung zum Tragen kämen. Es ist für den Fachmann ohne weiteres erkennbar, dass grundsätzlich über die Querverbinder-Zentralverbindung 70 zwei Querverbinder unterschiedlicher Ausführungsformen gekoppelt werden können.

### Bezugszeichen

- 100: Querverbinder
- 10: Längsstab
- 11: erster Oberflächenabschnitt
- 12: zweiter Oberflächenabschnitt
- 20: Querstrebe
- 30: Klemmkopf
- 31: Stabaufnahme
- 32: Lagerfläche
- 35: Langlochbohrung
- 39: Innenflächen
- 40: Klemmschraube
- 41: Drückelement
- 42: Klemmzahn
- 43: Zylinderbolzen
- 50: Gabelhalterung
- 51: Zylinderbohrung
- 60: Federelement
- 70: Querverbinder-Zentralverbindung
- A1: Schwenkachse
- B1: Drehachse
- S1: Wippenlagerung
- A2: Schwenkachse
- F2: Innenflächenabschnitt
- S2: Winkellagerung
- V: Lateralbewegung(svektor)
- X: Longitudinalrichtung
- Y: Vertikalrichtung
- Z: Transversalrichtung
- α: Winkelposition

## Patentansprüche

1. Medizinischer Querverbinder (100) umfassend:
- eine Querstrebe (20) zur Verbindung zweier vorzugsweise mit Pedikelschrauben oder dergleichen Knochenimplantaten in Wirkeingriff bringbarer Längsstäbe (10), insbesondere zur Lagepositionierung von Wirbeln einer Wirbelsäule, und
- zumindest einen an der Querstrebe (20) in einer Winkellagerung (S2) schwenkbar gelagerten Klemmkopf (30) mit einer muldenförmigen Stabaufnahme (31) und einer Klemmschraube (40),
wobei die Klemmschraube (40) bei deren Betätigung mittels eines über eine Wippenlagerung (S1) um eine Schwenkachse (A1) schwenkbar gelagerten Klemmzahns (42) eine Klemmkraft auf den zumindest einen in die Stabaufnahme (31) eingesetzten Längsstab (10) aufbringt und
dabei gleichzeitig oder zeitlich versetzt eine Winkelposition (α) der Winkellagerung (S2) zwischen dem Klemmkopf (30) und der Querstrebe (20) fixiert, **dadurch gekennzeichnet, dass**
die Wippenlagerung (S1) als eine schwimmende Lagerung ausgeführt ist,
wobei die schwimmende Lagerung eine Schwenkbewegung und eine infolge von Klemmkräften zwischen dem Längsstab (10) und dem Klemmzahn (42) erzeugte Lateralbewegung (V) der Schwenkachse (A1) des Klemmzahns (42) erlaubt,
welche durch Umlenken des Kraftflusses auf die Winkelposition (α) der Winkellagerung (S2) zwischen dem Klemmkopf (30) und der Querstrebe (20) fixierend und/oder verriegelnd einwirkt.

2. Medizinischer Querverbinder (100) nach Anspruch 1, wobei die Winkellagerung (S2) polyaxial ausgeführt ist, insbesondere in Form einer Kugelgelenk-Verbindung zwischen dem Klemmkopf (30) und der Querstrebe (20), so dass die Winkelposition (α) in zumindest zwei, vorzugsweise drei Achsrichtungen (X, Y, Z) veränderlich und/oder fixierbar ist.

3. Medizinischer Querverbinder (100) nach vorhergehendem Anspruch 2, wobei die polyaxiale Winkellagerung (S2) mittels eines Federelementes (60) vorgespannt und/oder vorspannbar ausgeführt ist.

4. Medizinischer Querverbinder (100) nach einem der vorhergehenden Ansprüche, wobei die Klemmschraube (40) reversibel lösbar und/oder selbstsichernd einschraubbar ist.

5. Medizinischer Querverbinder (100) nach einem der vorhergehenden Ansprüche, wobei der Klemmzahn (42) für unterschiedliche Durchmesser des Längsstabes (10) unterschiedlich ausgeformt und/oder dimensioniert einrichtbar und/oder eingerichtet ist, wobei weiter bevorzugt der Durchmesser für den Längsstab (10) 3 bis 7 mm beträgt, besonders bevorzugt im zervikalen bzw. den Hals betreffenden Bereich 3,5 bis 4,0 mm und/oder im lumbalen bzw. die Lendenwirbel betreffenden Bereich 5,5 bis 6,0 mm.

6. Medizinisches Produktset, vorzugsweise ein Pedikelschrauben- und -hakensystem zur Stabilisation bzw. Korrektur der Wirbelsäule, mit einem medizinischen Querverbinder (100) nach einem der Ansprüche 1 bis 4, in Kombination mit zumindest:
- einem Längsstab (10), vorzugsweise eine Vielzahl von Längsstäben (10), vorzugsweise in einer Zusammenstellung der Längsstäbe (10) von gerader und/oder gebogener Form und/oder nach unterschiedlichen Größen und/oder Härtegraden und/oder Materialien; und/oder
- einem Knochenimplantat wie einer Poly- und /oder Monoaxialschraube; und /oder
- einem Pedikelhaken; und /oder
- einem chirurgischen Instrument wie einer Stabeindrückzange, einem Implantathalter, einem Distraktor oder dergleichen; und/oder
- einem zugehörigen Drehmomentschlüssel.

## Claims

1. A medical cross connector (100) comprising:
- a cross strut (20) for connecting two longitudinal rods (10) which can be brought into operative engagement preferably with pedicle screws or similar bone implants, in particular for orientation of vertebrae of a spinal column, and
- at least one clamping head (30) being mounted pivotably on the cross strut (20) in an angular bearing (S2) and having a trough-shaped rod receptacle (31) and a clamping screw (40),
wherein the clamping screw (40), when actuated, applies a clamping force to the at least one longitudinal rod (10) inserted into the rod receptacle (31) by means of a clamping tooth (42) mounted pivotably about a pivot axis (A1) via a rocker bearing (S1), and
at the same time or with a time offset fixes an angular position (α) of the angular bearing (S2) between the clamping head (30) and the cross strut (20),
**characterized in that**
the rocker bearing (S1) is configured as a floating bearing,
wherein the floating bearing permits a pivoting movement and a lateral movement (V) of the pivot axis (A1) of the clamping tooth (42), wherein the lateral movement is generated due to clamping forces between the longitudinal rod (10) and the clamping tooth (42),
which acts in a fixing and/or locking manner on the angular position (α) of the angular bearing (S2) between the clamping head (30) and the cross strut (20) by reversing the force flow.

2. The medical cross connector (100) according to claim 1, wherein the angular bearing (S2) is configured to be polyaxial, in particular in the form of a ball joint connection between the clamping head (30) and the cross strut (20), so that the angular position (α) can be changed and/or fixed in at least two, preferably three axial directions (X, Y, Z).

3. The medical cross connector (100) according to preceding claim 2, wherein the polyaxial angular bearing (S2) is configured to be preloaded and/or preloadable by means of a spring element (60).

4. The medical cross connector (100) according to one of the preceding claims, wherein the clamping screw (40) is screwable in a reversibly releasable and/or in a self-locking manner.

5. The medical cross connector (100) according to one of the preceding claims, wherein the clamping tooth (42) is adjustable and/or adjusted to be differently shaped and/or dimensioned for different diameters of the longitudinal rod (10), wherein further preferably the diameter for the longitudinal rod (10) is 3 to 7 mm, particularly preferably 3.5 to 4.0 mm in the cervical or neck region and/or 5.5 to 6.0 mm in the lumbar or lumbar vertebrae region.

6. A medical product set, preferably a pedicle screw and hook system for stabilization and/or correction of the spinal column, comprising a medical cross connector (100) according to one of the claims 1 to 4, in combination with at least:
- a longitudinal rod (10), preferably a plurality of longitudinal rods (10), preferably in an assembly of the longitudinal rods (10) of straight and/or curved shape and/or according to different sizes and/or degrees of hardness and/or materials; and/or
- a bone implant such as a poly- and/or monoaxial screw; and/or
- a pedicle hook; and/or
- a surgical instrument such as a rod insertion forceps, an implant holder, a distractor or the like; and/or
- an associated torque wrench.

## Revendications

1. Connecteur transversal médical (100) comprenant :
- une entretoise transversale (20) destinée à la liaison de deux tiges longitudinales (10) pouvant être mises en prise de préférence avec des vis pédiculaires ou des implants osseux similaires, en particulier destinée au positionnement de vertèbres d'une colonne vertébrale, et
- au moins une tête de serrage (30) montée de manière pivotante au niveau de l'entretoise transversale (20) dans un palier angulaire (S2) avec un logement de tige (31) en forme de creux et une vis de serrage (40),
dans lequel la vis de serrage (40), lors de son actionnement au moyen d'une dent de serrage (42) montée de manière pivotante autour d'un axe de pivotement (A1), applique une force de serrage sur la au moins une tige longitudinale (10) insérée dans le logement de tige (31) par l'intermédiaire d'un palier à bascule (51) et
à cet effet fixe en même temps ou de manière décalée dans le temps une position angulaire (α) du palier angulaire (S2) entre la tête de serrage (30) et l'entretoise transversale (20), **caractérisé en ce que**
le palier à bascule (51) est réalisé en tant que palier flottant,
dans lequel le palier flottant permet un mouvement pivotant et un mouvement latéral (V) de l'axe pivotant (A1) de la dent de serrage (42) généré à la suite de forces de serrage entre la tige longitudinale (10) et la dent de serrage (42),
qui agit par le biais d'une réorientation du flux de force sur la position angulaire (α) du palier angulaire (S2) entre la tête de serrage (30) et l'entretoise transversale (20) par fixation et/ou verrouillage.

2. Connecteur transversal médical (100) selon la revendication 1, dans lequel le palier angulaire (S2) est réalisé de manière polyaxiale, en particulier sous la forme d'une liaison à articulation sphérique entre la tête de serrage (30) et l'entretoise transversale (20), de sorte que la position angulaire (α) est variable et/ou peut être fixée dans au moins deux, de préférence trois directions d'axe (X, Y, Z).

3. Connecteur transversal médical (100) selon la revendication 2 précédente, dans lequel le palier angulaire polyaxial (S2) est réalisé de manière prétendue et/ou apte à être prétendu au moyen d'un élément ressort (60).

4. Connecteur transversal médical (100) selon l'une quelconque des revendications précédentes, dans lequel la vis de serrage (40) peut être libérée de manière réversible et/ou vissée de manière autobloquante.

5. Connecteur transversal médical (100) selon l'une quelconque des revendications précédentes, dans lequel la dent de serrage (42) est façonnée et/ou dimensionnée de manière configurable et/ou configurée différemment pour différents diamètres de la tige longitudinale (10), dans lequel en outre plus préférentiellement le diamètre pour la tige longitudinale (10) est de 3 à 7 mm, le plus préférentiellement dans la plage cervicale ou concernant le cou de 3,5 à 4,0 mm et/ou dans la plage concernant la colonne lombaire ou les vertèbres lombaires de 5,5 à 6,0 mm.

6. Ensemble de produits médicaux, de préférence une vis pédiculaire et un système à crochets destinés à la stabilisation ou la correction de la colonne vertébrale, avec un connecteur transversal médical (100) selon l'une quelconque des revendications 1 à 4, en combinaison avec au moins :
- une tige longitudinale (10), de préférence une pluralité de tiges longitudinales (10), de préférence dans une combinaison des tiges longitudinales (10) de forme droite et/ou courbée et/ou selon des grandeurs et/ou des degrés de dureté et/ou des matériaux différents ; et/ou
- un implant osseux tel qu'une vis polyaxiale et/ou monoaxiale ; et/ou
- un crochet pédiculaire ; et/ou
- un instrument chirurgical tel qu'une languette d'empreinte de tige, un support d'implant, un distracteur ou similaire ; et/ou
- une clé dynamométrique correspondante.
